Europäisches Patentamt

European Patent Office

Office européen des brevets

(11) Veröffentlichungsnummer : **0 126 999**
**B1**

# EUROPÄISCHE PATENTSCHRIFT

(45) Veröffentlichungstag der Patentschrift :
03.12.86

(51) Int. Cl.⁴ : **C 07 C 103/19**

(21) Anmeldenummer : **84104877.0**

(22) Anmeldetag : **02.05.84**

(54) **Verfahren zur Herstellung von sphärisch-kristallinem 5-(Hydroxy)-tetracyclinhydrochlorid (Oxytetracyclinhydrochlorid).**

(30) Priorität : 02.05.83 HU 149583

(43) Veröffentlichungstag der Anmeldung :
05.12.84 Patentblatt 84/49

(45) Bekanntmachung des Hinweises auf die Patenterteilung : 03.12.86 Patentblatt 86/49

(84) Benannte Vertragsstaaten :
AT BE CH DE FR GB IT LI NL SE

(56) Entgegenhaltungen :
US-A- 2 867 661
"Organikum: - organisch-chemisches Grundpraktikum", 9. überarbeitete Auflage, 1969, VEB DEUTSCHER VERLAG DER WISSENSCHAFTEN, Berlin, Seiten 62-64 "Azeotrope Destillation"

(73) Patentinhaber : BIOGAL GYOGYSZERGYAR
Pallagi u. 13.
H-4042 Debrecen (HU)

(72) Erfinder : Szarvas, Miklòs, Dr. Dipl.-chem.
Péterfia u. 40
H-4042 Debrecen (HU)
Erfinder : Horváth, Eva, geb. Fehér, Dr. Dipl.-Chem.
Naphegy u. 33, II. 5
H-1016 Budapest (HU)
Erfinder : Cséke, Làszlò, Dipl.-chem.
Hajò u. 4
H-4042 Debrecen (HU)
Erfinder : Bálint, János, Dr. Dipl.-chem.
Péchy u. 5
H-4042 Debrecen (HU)
Erfinder : Fábián, Ferenc
Hollò J. u. 4
H-4042 Debrecen (HU)
Erfinder : Kun, Lajos
Szegfü u. 6
H-4042 Debrecen (HU)

(74) Vertreter : Beszédes, Stephan G. Dr.
Münchener Strasse 80a Postfach 1168
D-8060 Dachau (DE)

## Beschreibung

Die Erfindung betrifft ein neues Verfahren zur Herstellung von sphärisch-kristallinem 5-(Hydroxy)-tetracyclinhydrochlorid [Oxytetracyclinhydrochlorid], welches auch im technischen beziehungsweise industriellen Maßstab einfach durchführbar ist.

Das 5-(Hydroxy)-tetracyclinhydrochlorid [Oxytetracyclinhydrochlorid] ist ein der Gruppe der Tetracycline angehörendes Antibioticum mit breitem Wirkungsspektrum. Es findet einerseits in der Human- und Veterinärmedizin und andererseits als Zwischenprodukt bei der Herstellung hochreiner 5-(Hydroxy)-tetracyclinderivate [Oxytetracyclinderivate] Verwendung.

Das 5-(Hydroxy)-tetracyclinhydrochlorid [Oxytetracyclinhydrochlorid] zeigt im festen Zustand Polymorphie, das heißt, daß von ihm mehrere Kristallformen bekannt sind. Die eine Form besteht aus langen nadelförmigen Kristaller und die andere aus orthorhombischen Plättchen. Die letztere Form ist eine dichte Kristallmasse, die kein Wasser enthält. (Ann. New York Acad. Sci. 53 [1950-51], 229 ; J. Am. Chem. Soc. 73 [1951], 4 211 ; 74 [1952], 841) und bei Kristallisieren aus entsprechend konzentrierten Lösungen durch zusammenwachsen der Kristallflächen Teilchen von sphärischer Form bildet.

Dieses aus sphärischen Teilchen bestehende Produkt ist für Arzneimittel geeigneter als das aus nadelförmigen Kristallen bestehende. Im Gegensatz zum letzteren ist das sphärische 5-(Hydroxy)-tetracyclinhydrochlorid [Oxytetracyclinhydrochlorid] nicht hygroskopisch und lädt sich nicht elektrostatisch auf, das heißt seine Handhabung und Dosierung ist wesentlich einfacher. Der Wassergehalt des sphärischen 5-(Hydroxy)-tetracyclinhydrochlorides [Oxytetracyclinhydrochlorides] kann durch einfaches Trocknen schnell auf einen Wert unterhalb der in den Arzneimittelbüchern vorgeschriebenen 2 Gew.-% gesenkt werden, während das nadelkristalline Produkt bei 50 °C unter Vakuum längere Zeit getrocknet werden muß. Die Kugelform der Teilchen hat ferner den Vorteil, daß das Verhältnis von Oberfläche zu Volumen den kleinsten Wert hat. Dadurch ist der Einfluß schädigender Faktoren, wie Feuchtigkeit und Licht, geringer.

Das aus nadelförmigen Kristallen bestehende 5-(Hydroxy)-tetracyclinhydrochlorid [Oxytetracyclinhydrochlorid] wird nach bekannten Verfahren in der Weise hergestellt, daß der das 5-(Hydroxy)-tetracyclin [Oxytetracyclin] enthaltenden Lösung Salzsäure (britische Patentschriften 718 020 und 718 032 sowie US-PS 2 516 080), mit Chlorwasserstoffgas gesättigte aliphatische Alkohole (belgische Patentschriften 632 331 und 638 881), methanolische Erdalkalichloridlösungen (US-PS 2 658 078 und 2 873 276, britische Patentschrift 718 027 und ungarische Patentschrift 160 457) oder langkettige quaternäre Alkylammoniumchloride (US-Patentschrift 2 867 661) zugesetzt wird beziehungsweise werden.

Das nach diesen Verfahren herstellbare nadelkristalline Oxytetracyclinhydrochlorid enthält etwa 6 bis 11 Gew.-% Kristallwasser. Es ist infolge seines hygroskopischen Charakters und seiner elektrostatischen Eigenschaften schlecht zu handhaben, nicht gut dosierbar und haftet an den Vorrichtungen, wodurch Verluste entstehen. Durch seinen Wassergehalt ist das Produkt weniger stabil.

Gemäß der US-PS 2 867 661 kann ein nicht hygroskopisches Produkt erhalten werden, wenn das 5-(Hydroxy)-tetracyclinhydrochlorid [Oxytetracyclinhydrochlorid] in einem organischen Lösungsmittelmedium aus Aceton, Benzaldehyd, Furfural, aliphatischen Alkoholen mit 2 bis 4 Kohlenstoffatomen, oder niederen Alkyläthern von Äthylenglykol oder Gemischen dieser Lösungsmittel auf Temperaturen von etwa 50 °C bis zur Siedetemperatur unter Rückfluß erhitzt wird. Das auf diese Weise erhaltene 5-(Hydroxy)-tetracyclinhydrochlorid [Oxytetracyclinhydrochlorid] ist weniger hygroskopisch, enthält jedoch immer noch nicht genügend Teilchen mit Kugelgestalt.

Durch Kristallisieren des durch Gärung gewonnenen 5-(Hydroxy)-tetracyclinhydrochlorides [Oxytetracyclinhydrochlorides] aus einem Gemisch, bestehend aus einem aliphatischen Alokohol mit 1 bis 4 Kohlenstoffatom(en), einem aliphatischen Keton und konzentrierter Salzsäure, kann gemäß der polnischen Patentschrift 49 215 ein sphärisches 5-(Hydroxy)-tetracyclinhydrochlorid [Oxytetracyclinhydrochlorid] mit einem Raumgewicht von mehr als 0,6 kg/l [0,6 kg/dm$^3$] hergestellt werden. Dieses ist zur Zubereitung von Arzneimitteln geeignet. Der Nachteil dieses Verfahrens ist die Notwendigkeit, ein ternäres Lösungsmittelgemisch verwenden zu müssen.

Der Erfindung liegt die Aufgabe zugrunde, ein einfaches, wirtschaftliches und technisch beziehungsweise industriell durchführbares Verfahren zur Herstellung von sphärisch-kristallinem 5-(Hydroxy)-tetracyclinhydrochlorid [Oxytetracyclinhydrochlorid], durch welches aus dem Rohprodukt in hoher Ausbeute ein sehr reines sphärisches 5-(Hydroxy)-tetracyclinhydrochlorid [Oxytetracyclinhydrochlorid] erhalten werden kann, zu schaffen.

Das Obige wurde überraschenderweise durch die Erfindung erreicht.

Es wurde nämlich nunüberraschenderweise festgestellt, daß aus dem nadelförmige Kristalle aufweisenden 5-(Hydroxy)-tetracyclinhydrochlorid [Oxytetracyclinhydrochlorid] ein wasserfreies sphärisches 5-(Hydroxy)-tetracyclinhydrochlorid [Oxytetracyclinhydrochlorid] erhalten werden kann, wenn das Rohprodukt in einem aus 1 oder mehr Ester(n) von Essigsäure mit einem aliphatischen Alkohol bestehenden Lösungsmittel als mit Wasser ein azeotropes Gemisch bildendem Lösungsmittel zum Sieden erhitzt und gegebenenfalls ein Teil des Lösungsmittels azeotrop abdestilliert wird sowie das aus dem abgekühlten Gemisch auskristallisierte sphärische 5-(Hydroxy)-tetracyclinhydrochlorid [Oxytetracyclinhydrochlorid] abgetrennt und getrocknet wird.

Gegenstand der Erfindung ist daher ein Verfahren zur Herstellung von sphärisch-kristallinem 5-(Hydroxy)-tetracyclinhydrochlorid [Oxytetracyclinhydrochlorid] durch Erhitzen von nadelförmig kristallisiertem rohem trockenem oder filterfeuchtem 5-(Hydroxy)-tetracyclinhydrochlorid [Oxytetracyclinhydrochlorid] in einem mit Wasser ein azeotropes Gemisch bildenden Lösungsmittel zum Sieden sowie Abtrennen und Trocknen des aus dem abgekühlten Gemisch auskristallisierten sphärischen 5-(Hydroxy)-tetracyclinhydrochlorides [Oxytetracyclinhydrochlorides], welches dadurch gekennzeichnet ist, daß als mit Wasser ein azeotropes Gemisch bildendes Lösungsmittel ein Ester von Essigsäure mit einem aliphatischen Alkohol mit 1 bis 4 Kohlenstoffatom(en) oder Gemisch von solchen verwendet wird.

Vorzugsweise wird als Ester von Essigsäure mit einem aliphatischen Alkohol mit 1 bis 4 Kohlenstoffatom(en) Äthylacetat verwendet.

Vorteilhaft werden 2 bis 6 Vol.-Teile, insbesondere 3 bis 5 Vol.-Teile, ganz besonders 4 Vol.-Teile, Lösungsmittel je Gew.-Teil-5-(Hydroxy)-tetracyclinhydrochlorid [Oxytetracyclinhydrochlorid] eingesetzt.

Gegebenenfalls wird vor dem Abkühlen des Gemisches zum Auskristallisieren des 5-(Hydroxy)-tetracyclinhydrochlorides [Oxytetracyclinhydrochlorides] ein Teil des Wassers mit Lösungsmittel azeotrop abdestilliert.

Nach einer zweckmäßigen Ausführungsform des erfindungsgemäßen Verfahrens wird wie folgt vorgegangen. Das nadelkristalline Rohprodukt wird in einem Alkylacetat suspendiert. Die Suspension wird 10 bis 60 Minuten lang bei 55 bis 125 °C gerührt beziehungsweise geschüttelt, wobei das gesamte Salz 5-(Hydroxy)-tetracyclinhydrochlorid [Oxytetracyclinhydrochlorid] in die gewünschte Kristallform übergeht und praktisch wasserfrei wird. Das Kristallwasser des 5-(Hydroxy)-tetracyclinhydrochlorides [Oxytetracyclinhydrochlorides] bildet mit dem Lösungsmittel ein azeotropes Gemisch und das 5-(Hydroxy)-tetracyclinhydrochlorid [Oxytetracyclinhydrochlorid] ist gezwungen, ohne Kristallwasser zu kristallisieren. Dabei werden optimale Ergebnisse erzielt, wenn das aus dem Lösungsmittel und dem Wasser gebildete Gemisch 1 bis 10 Gew.-% Wasser enthält. Die chemischen Verunreinigungen, die stärker apolar als das 5-(Hydroxy)-tetracyclinhydrochlorid [Oxytetracyclinhydrochlorid] sind, lösen sich im Alkylacetat, was an dessen dunkler werdender Farbe erkennbar ist.

Im allgemeinen ist es ausreichend, das Gemisch abzukühlen, um die Kristallisation auszulösen. Wenn jedoch ein filterfeuchtes Rohprodukt verwendet wurde, bildet auch das anhaftende Wasser ein azeotropes Gemisch. In diesem Fall ist es zweckmäßig, einen Teil des Wassers in Form des azeotropen Gemisches abzudestillieren, bevor kristallisiert wird. Das ausfallende 5-(Hydroxy)-tetracyclinhydrochlorid [Oxytetracyclinhydrochlorid] weist einen Wirkstoffgehalt von 99 bis 100 Gew.-% auf und enthält weniger als 1 Gew.-% Wasser.

Das sphärische 5-(Hydroxy)-tetracyclinhydrochlorid [Oxytetracyclinhydrochlorid] ist gut schüttfähig, freifließend und gut mischbar. Diese Eigenschaften sind für die Herstellung der verschiedenen Zubereitungen, wie Kapseln und Pulverampullen, wertvoll, weil sie das Bereiten des homogenisierten Pulvergemisches erleichtern. Die Verluste bei der automatischen Dosierung sind geringer.

Es ist überraschend, daß gerade mit den erfindungsgemäß verwendeten Lösungsmitteln sphärisch-kristallines 5-(Hydroxy)-tetracyclinhydrochlorid [Oxytetracyclinhydrochlorid] mit einem besonders hohen Anteil an Teilchen mit Kugelgestalt und besonders geringem Wassergehalt sowie besonders geringer Hygroskopizität und damit besonders hoher Reinheit erhalten werden kann. Ferner ist es überraschend, daß durch die erfindungsgemäß verwendeten Lösungsmittel der Wassergehalt des Gemisches aus Lösungsmittel und Wasser besonders gut in den optimalen Bereich eingestellt werden kann, wobei, falls er sich von Haus aus zu hoch einstellen würde, wie insbesondere im Falle der Verwendung von filterfeuchtem nadelförmig kristallisiertem rohem 5-(Hydroxy)-tetracyclinhydrochlorid [Oxytetracyclinhydrochlorid] als Ausgangsmaterial, mit den erfindungsgemäß verwendeten Lösungsmitteln eine günstige Abdestillation des Wassers in Form von azeotropen Gemischen desselben mit den Lösungsmitteln unter passend schneller Erzielung eines optimalen Wassergehaltes des Gemisches aus Lösungsmittel und Wasser ermöglicht ist. Dazu vermochte der Stand der Technik keine Anregung zu geben, da gemäß der US-PS 2 867 661 das Erhitzen zum Sieden unter Rückfluß durchgeführt wird, also von einem azeotropen Abdestillieren des Wassers mit dem Lösungsmittel und damit dem planmäßigen Steuern des Wassergehaltes des Gemisches aus Lösungsmittel und Wasser unter Erzielung von optimalen Werten abgelenkt wurde, indem so gearbeitet wurde, daß dies unmöglich ist. Damit wurde in der US-PS 2 867 661, auch wenn rein zufällig ein mit Wasser ein azeotropes Gemisch bildendes Lösungsmittel verwendet wurde, diese Eigenschaft, von welcher nicht einmal andeutungsweise die Rede ist, überhaupt nicht, geschweige denn im Sinne der Erfindung zunutze gemacht.

Die Erfindung wird an Hand der folgenden Beispiele näher erläutert.

Beispiel 1

Es wurden 20 g trockenes nadelkristallines 92 gew.-%-iges 5-(Hydroxy)-tetracyclinhydrochlorid [Oxytetracyclinhydrochlorid] in 80 ml Äthylacetat suspendiert. Das Gemisch wurde auf dem Wasserbad unter Rühren beziehungsweise Schütteln zum Sieden erhitzt und 30 Minuten lang am Sieden gehalten. Bereits aus der heißen Lösung fielen lebhaft gelbe Kristalle von 5-(Hydroxy)-tetracyclinhydrochlorid [Oxytetracyclinhydrochlorid] aus. Das Gemisch wurde auf Raumtemperatur gekühlt. Die Kristalle wurden abfiltriert, mit 25 ml Äthylacetat gewaschen und bei 50 °C an der Luft getrocknet.

So wurden 18,16 g (98,8 % der Theorie) sphärisches 5-(Hydroxy)-tetracyclinhydrochlorid [Oxytetracyclinhydrochlorid], das 99,3 Gew.-% Wirkstoff und 0,4 Gew.-% Wasser enthielt, erhalten. Das Produkt entsprach den Qualitätsvorschriften des britischen Arzneimittelbuches von 1980 (B.P. 80) sowie denen des 1980 herausgegebenen Arzneimittelbuches der Vereinigten Staaten (USP XX). Es hatte in mit 1/10 m Salzsäure bereiteter 1 vol.-%-iger Lösung bei 20 °C nach 6 Minuten langem Stehenlassen nach der Zubereitung ein spezifisches Dehnungsvermögen von — 190 bis — 197° (zum Vergleich das nach dem ungarischen Arzneibuch nach derselben Meßverfahrensweise : — 188 bis — 200° [nach dem deutschen Arzneibuch : ebenfalls — 188 bis — 200°]). Bei einer Luftfeuchtigkeit von 71 bis 90 % (gemessen in einem Hygrostat [einem geschlossenen Raum, in welchem die Luftfeuchtigkeit präzis eingestellt werden kann]) hat es innerhalb 24 Stunden kein Wasser angezogen.

Beispiel 2

Es wurden 20 g trockenes nadelkristallines 92 gew.-%-iges 5-(Hydroxy)-tetracyclinhydrochlorid [Oxytetracyclinhydrochlorid] in 80 ml n-Butylacetat suspendiert. Das Gemisch wurde auf dem Wasserbad unter Rühren beziehungsweise Schütteln zum Sieden erhitzt und 1/2 Stunde lang am Sieden gehalten. Das Kristallisieren und Abtrennen erfolgten auf die im Beispiel 1 angegebene Weise.

So wurden 17,82 g (96,7 % der Theorie) sphärisches 5-(Hydroxy)-tetracyclinhydrochlorid [Oxytetracyclinhydrochlorid], welches 99,1 Gew.-% Wirkstoff und 0,8 Gew.-% Wasser enthielt, erhalten. Die sonstigen Parameter stimmten mit denen des Produktes des Beispieles 1 überein.

Beispiel 3

Es wurde ein 20 g 5-(Hydroxy)-tetracyclinhydrochloridwirkstoff [Oxytetracyclinwirkstoff] als Rohprodukt enthaltender filterfeuchter Filterkuchen in 80 ml Äthylacetat suspendiert. Die Suspension wurde bis zum Sieden erhitzt und 30 Minuten lang auf dem Wasserbad bei 80 °C gerührt beziehungsweise geschüttelt. Während dieser Zeit wurden 20 ml azeotropes Gemisch abdestilliert. Dann wurde das Gemisch auf Raumtemperatur gekühlt und die Kristalle wurden abfiltriert, mit 25 ml Äthylacetat gewaschen und bei 50 °C an der Luft getrocknet.

So wurden 18,8 g (94 % der Theorie) sphärisches 5-(Hydroxy)-tetracyclinhydrochlorid [Oxytetracyclinhydrochlorid], das 99,8 Gew.-% Wirkstoff und 0,2 Gew.-% Wasser enthielt, erhalten. Die sonstigen Parameter stimmten mit denen des Produktes des Beispieles 1 überein.

**Patentansprüche**

1. Verfahren zur Herstellung von sphärischkristallinem 5-(Hydroxy)-tetracyclinhydrochlorid [Oxytetracyclinhydrochlorid] durch Erhitzen von nadelförmig kristallisiertem rohem trockenem oder filterfeuchtem 5-(Hydroxy)-tetracyclinhydrochlorid [Oxytetracyclinhydrochlorid] in einem mit Wasser ein azeotropes Gemisch bildenden Lösungsmittel zum Sieden sowie Abtrennen und Trocknen des aus dem abgekühlten Gemisch auskristallisierten sphärischen 5-(Hydroxy)-tetracyclinhydrochlorides [Oxytetracyclinhydrochlorides], dadurch gekennzeichnet, daß man als mit Wasser ein azeotropes Gemisch bildendes Lösungsmittel einen Ester von Essigsäure mit einem aliphatischen Alkohol mit 1 bis 4 Kohlenstoffatom(en) oder Gemisch von solchen verwendet.

2. Verfahren nach Anspruch 1, dadurch gekennzeichnet, daß man als Ester von Essigsäure mit einem aliphatischen Alkohol mit 1 bis 4 Kohlenstoffatom(en) Äthylacetat verwendet.

3. Verfahren nach Anspruch 1 oder 2, dadurch gekennzeichnet, daß man vor dem Abkühlen des Gemisches zum Auskristallisieren des 5-(Hydroxy)-tetracyclinhydrochlorides [Oxytetracyclinhydrochlorides] einen Teil des Wassers mit Lösungsmittel azeotrop abdestilliert.

**Claims**

1. A process for preparing spherically cristalline 5-(hydroxy)-tetracycline hydrochloride [oxytetracycline hydrochloride] by heating to boiling needle-shaped cristallized raw dry or filter-wet 5-(hydroxy)-tetracycline hydrochloride [oxytetracycline hydrochloride] in a solvent forming with water an azeotropic mixture, and separating and drying of the cristallized spherical 5-(hydroxy)-tetracycline hydrochloride [oxytetracycline hydrochloride] cristallized from the cooled mixture, characterized in that as solvent forming with water an azeotropic mixture an ester of acetic acid with an aliphatic alcohol having 1 to 4 carbon atom(s) or a mixture thereof is used.

2. A process according to claim 1, characterized in that as ester of acetic acid with an aliphatic alcohol having 1 to 4 carbon atom(s) ethyl acetat is used.

3. A process according to claim 1 or 2, characterized in that prior to a cooling of the mixture for cristallizing 5-(hydroxy)-tetracycline hydrochloride [oxytetracycline hydrochloride] a part of the water is destilled azeotropically with solvent.

**Revendications**

1. Procédé de préparation de chlorhydrate de 5-hydroxytétracycline (chlorhydrate d'oxytétracycline) par chauffage à ébullition de chlorhydrate de 5-hydroxytétracycline (chlorhydrate d'oxytétracycline) sec, brut ou humide de filtre, cristallisé en aiguilles dans un solvant formant avec l'eau un mélange azéotropique ainsi que séparation et séchage du chlorhydrate de 5-hydroxytétracycline (chlorhydrate d'oxytétracycline) sphérocristallin du mélange refroidi, caractérisé en ce que l'on emploie comme solvant formant un mélange azéotrope avec l'eau un ester de l'acide acétique avec un alcool aliphatique renfermant de 1 à 4 atomes de carbone ou un mélange de ces esters.

2. Procédé selon la revendication 1, caractérisé en ce que l'on emploie comme ester de l'acide acétique avec un alcool aliphatique renfermant de 1 à 4 atomes de carbone l'acétate d'éthyle.

3. Procédé selon la revendication 1 ou 2, caractérisé en ce que, avant le refroidissement du mélange pour la cristallisation du chlorhydrate de 5-hydroxytétracycline (chlorhydrate d'oxytétracycline), on élimine une partie de l'eau par distillation azéotrope en présence du solvant.